# EUROPEAN PATENT APPLICATION

(11) **EP 4 414 373 A1**
(43) Date of publication of application: **14.08.2024**
(21) Application number: 22878477.3
(22) Date of filing: 03.10.2022
(51) Int. Cl.: C07H 23/00, A61K 31/7135, A61K 33/242, A61P 35/00

(54) **GLUCOSE DERIVATIVES AND ANTICANCER AGENT USING SAME**

(30) Priority: 06.10.2021 JP 2021165100
(71) Applicant: Orbio Corporation, Kyoto-shi, Kyoto 602-0841 (JP)
(72) Inventor: YAMAGUCHI, Eiichi, Tokyo 112-0001 (JP); YAMADA, Katsuya, Hirosaki-shi, Aomori 036-8560 (JP); ONO, Koki, Hirosaki-shi, Aomori 036-8560 (JP); TANAKA, Kota, Hirosaki-shi, Aomori 036-8560 (JP); IIJIMA, Hideki, Otsu-shi, Shiga 520-0047 (JP)
(74) Representative: Mewburn Ellis LLP
(86) International application number: PCT/JP2022/037001
(87) International publication number: WO 2023/058608

(57) **Abstract**

Provided are novel glucose derivatives and an anticancer agent using the same. The glucose derivatives according to the present invention consist of an L-glucose derivative represented by general formula (1) and a D-glucose derivative represented by general formula (2). In formula (1): X^{L2} represents an -SAuR^{L2} group; and X^{L1}, X^{L3}, X^{L4} and X^{L5} independently represent an -OR^{L1} group, an -NH₂ group or a fluorine atom. R^{L2} represents a ligand, and R^{L1} represents a hydrogen atom or an organic group. In formula (2): X^{D2} represents an -SAuR^{D2} group; and X^{D1}, X^{D3}, X^{D4} and X^{D5} independently represent an -OR^{D1} group, an -NH₂ group or a fluorine atom. R^{D2} represents a ligand, and R^{D1} represents a hydrogen atom or an organic group. The anticancer agent according to the present invention comprises at least one of the aforesaid glucose derivatives.

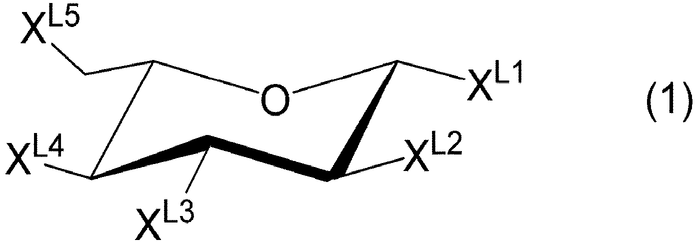

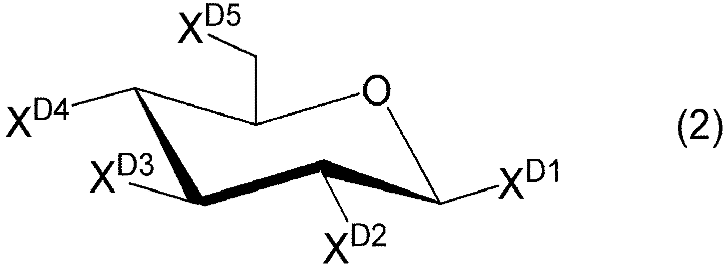

## Description

### TECHNICAL FIELD

The present invention relates to a glucose derivative and an anticancer agent using the glucose derivative.

### BACKGROUND ART

As one of common methods for treating cancer, use of an anticancer agent has been known. Generally, the anticancer agent acts not only on cancer cells but also on normal cells, resulting in a strong side effect. How to reduce such a side effect has been a challenge in cancer treatment. As a countermeasure against the above challenge, a molecular targeted drug that identifies a molecule specifically expressed by cancer cells and suppresses growth and metastasis of the cancer cells has attracted attention. The molecular target drug is expected to suppress a side effect caused by a classical anticancer agent. However, in reality, a molecule purported to be specifically expressed by cancer cells is also expressed by normal cells, and the molecular target drug also acts on different molecules than expected, resulting in no small number of side effects. Needless to say, the human body is a collection of cells. Being cells, they require a carbon source for energy and growth. Therefore, a method of identifying cancer by labeling a molecule to be taken up by cells, rather than by targeting a specific protein or molecule in our organism, has recently drawn renewed attention. (Non-Patent Document 1).

### Citation List

### Non-Patent Document

Non-Patent Document 1: Ono K. et al., Cancers, 12: 850, 2020

### DISCLOSURE OF THE INVENTION

### Problems to be Solved by the Invention

An object of the present invention is to provide a new glucose derivative and an anticancer agent using the glucose derivative.

### Means for Solving the Problems

The present inventors conducted extensive studies to solve the above problem. As a result, the present inventors have found a novel glucose derivative that is taken up by cancer cells and is toxic to cancer cells. Thus, the present invention has been completed. Specifically, the present invention provides the following aspects.

A first aspect of the present invention relates to a glucose derivative selected from the group consisting of an L-glucose derivative represented by General Formula (1) below and a D-glucose derivative represented by General Formula (2) below: 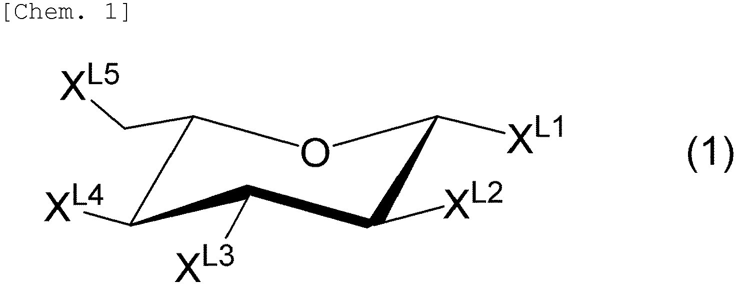
where X^{L2} denotes an -SAuR^{L2} group, X^{L1}, X^{L3}, X^{L4}, and X^{L5} each independently denote an -OR^{L1} group, an -NH₂ group, or a fluorine atom,
R^{L2} denotes a ligand, and R^{L1} denotes a hydrogen atom or an organic group.

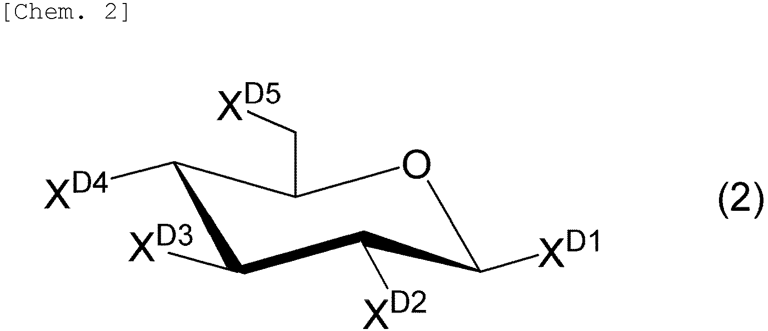

where X^{D2} denotes an -SAuR^{D2} group, X^{D1}, X^{D3}, X^{D4}, and X^{D5} each independently denote an -OR^{D1} group, an -NH₂ group, or a fluorine atom,
R^{D2} denotes a ligand, and R^{D1} denotes a hydrogen atom or an organic group.

The glucose derivative according to the first aspect of the present invention includes a gold atom (Au) in its molecule and the gold atom may be significantly toxic to cancer cells when taken up by the cancer cells.

A second aspect of the present invention relates to an anticancer agent comprising at least one of the glucose derivatives according to the first aspect of the present invention.

### Effects of the Invention

The present invention can provide a new glucose derivative and an anticancer agent using the glucose derivative. The glucose derivative is taken up by cancer cells and is toxic to the cancer cells.

### BRIEF DESCRIPTION OF THE DRAWINGS

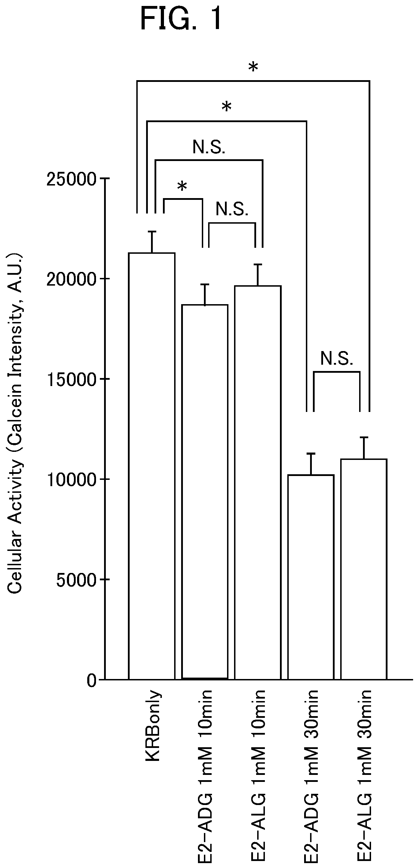

Fig. 1 shows results of an activity of viable cells when E2-ADG or E2-ALG at a concentration of 1,000 µM was administrated to mouse insulinoma MIN6 cells (Miyazaki J. et al., Endocrinology 127: 126-132, 1990) at 5 DIV (days in vitro) of culture for 10 or 30 minutes, as assessed by a fluorescence intensity of a fluorescent marker Calcein. The error bar indicates standard deviation. All * indicate p < 0.001 (Bonferroni-Dunn test);

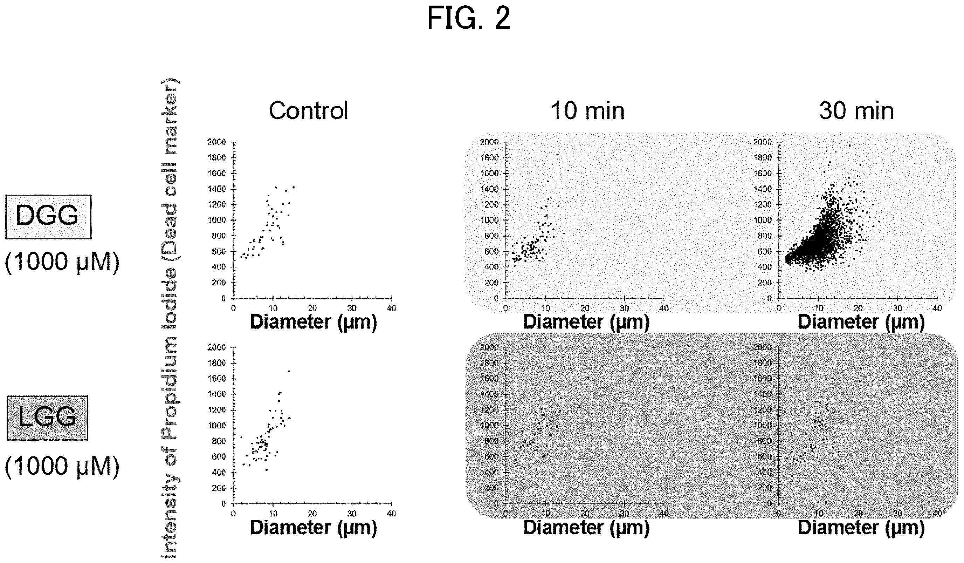

Fig. 2 shows results of occurrence of cell death when a D-glucose analog DGG and an L-glucose analog LGG, each in which a hydrogen atom in an OH group at position 1 of glucose was substituted with an -SAuP(C₂H₅)₃ group, at a concentration of 1,000 µM were administered to MIN6 cells at 5 DIV for 10 or 30 minutes, as assessed by an intensity of a dead cell marker Propidium Iodide (hereinafter referred to as "PI") positive signal. The horizontal axis indicates a diameter of an individual cell;

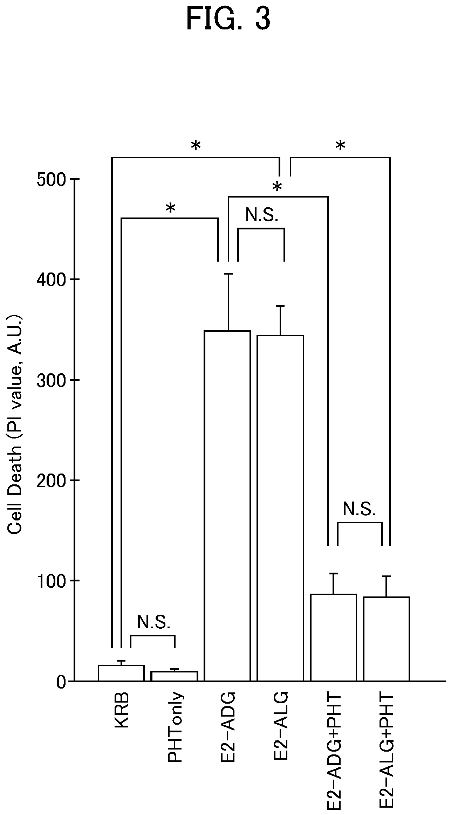

Fig. 3 shows a proportion of dead cells expressed as a PI value when E2-ADG or E2-ALG at a concentration of 1,000 µM was administrated to MIN6 cells at 11 DIV of culture in the presence or absence of 150 µM phloretin (hereafter referred to as "PHT") for 90 min. The error bar indicates standard deviation. All * indicate p < 0.001 (Bonferroni-Dunn test);

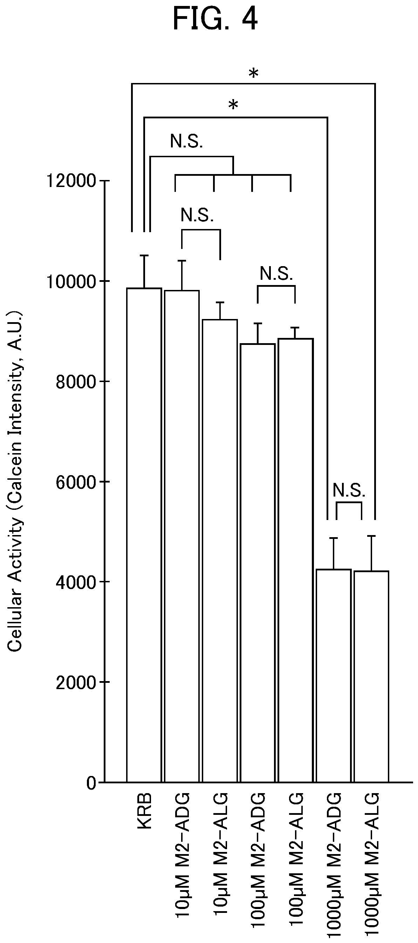

Fig. 4 shows results of an activity of viable cells when M2-ADG or M2-ALG at the indicated concentration was administrated to MIN6 cells at 4 DIV of culture for 30 minutes, as assessed by a fluorescence intensity of a fluorescent marker Calcein. The error bar indicates standard deviation. All * indicate p < 0.001 (Bonferroni-Dunn test); and

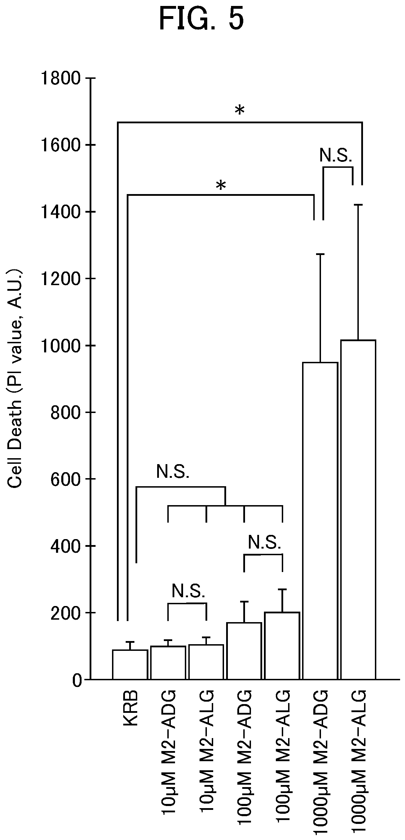

Fig. 5 shows a proportion of dead cells expressed as a PI value when M2-ADG or M2-ALG at the indicated concentration was administrated to MIN6 cells at 4 DIV of culture for 30 minutes. The error bar indicates standard deviation. All * indicate p < 0.001 (Bonferroni-Dunn test).

### PREFERRED MODE FOR CARRYING OUT THE INVENTION

Suitable embodiments of the present invention will be described in detail. However, the present invention is not limited to the following embodiments. Note that, correspondence between an abbreviation and a compound name used in the specification, including examples, is shown below.
Ac: acetyl group
Ac₂O: acetic anhydride
AcONa: sodium acetate
DBU: diazabicycloundecene
DCM: dichloromethane
DMF: N,N-dimethylformamide
Et: ethyl group
Et₃PAuCl: chloro(triethylphosphine)gold(I)
HClO₄: perchloric acid
HOBt: 1-hydroxybenzotriazole
IPE: diisopropyl ether
KSAc: S-potassium thioacetate
Me: methyl group
MeCN: acetonitrile
MeOH: methanol
Me₃PAuCl: chloro(trimethylphosphine)gold(I)
NaOMe: sodium methoxide
NIS: N-iodosuccinimide
PBr₃: phosphorus tribromide
Piv: pivaloyl group
PivCl: pivaloyl chloride
TCEP·HCl: tris(2-carboxyethyl)phosphine hydrochloride
Tf: trifluoromethanesulfonyl group
Tf₂O: trifluoromethanesulfonic anhydride
Trt: trityl group
TrtCl: trityl chloride

A glucose derivative according to the present embodiment has a structure represented by General Formula (1) below or a structure represented by General Formula (2) below.

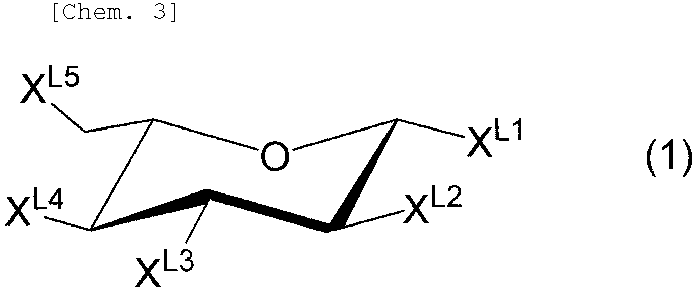

In Formula (1), X^{L2} denotes an -SAuR^{L1} group, X^{L1}, X^{L3}, X^{L4}, and X^{L5} each independently denote an -OR^{L1} group, an -NH₂ group, or a fluorine atom,
R^{L2} denotes a ligand, and R^{L1} denotes a hydrogen atom or an organic group.

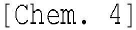

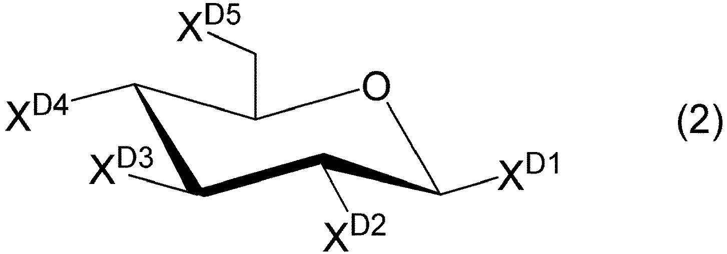

In Formula (2), X^{D2} denotes an -SAuR^{D2} group, X^{D1}, X^{D3}, X^{D4}, and X^{D5} each independently denote an -OR^{D1} group, an -NH₂ group, or a fluorine atom,
R^{D2} denotes a ligand, and R^{D1} denotes a hydrogen atom or an organic group.

The glucose derivative includes a D-form and an L-form, the structure represented by General Formula (1) is an L-glucose derivative and the structure represented by General Formula (2) is a D-glucose derivative. Furthermore, the glucose derivative may include anomeric isomers, that is, an anomer in which X^{L1} or X^{D1} is equatorially oriented and an anomer in which X^{L1} or X^{D1} is axially oriented. The L-glucose derivative or the D-glucose derivative according to the present embodiment may be a mixture thereof.

Examples of the ligand for R^{L2} or R^{D2} include a phosphine ligand, a thiolate ligand, an olefin ligand, or the like. R^{L2} or R^{D2} is preferably a phosphine ligand, more preferably a trialkylphosphine ligand, further more preferably a trimethylphosphine ligand or a triethylphosphine ligand, and particularly preferably a triethylphosphine ligand.

Examples of the organic group for R^{L1} or R^{D1} include an alkyl group, an alkenyl group, an alkynyl group, an aryl group, an acyl group, or the like. These groups may further have another substituent. A number of carbons in the organic group may be, for example, 1 to 10, preferably 1 to 5, and more preferably 1 to 3.

R^{L1} or R^{D1} is preferably a hydrogen atom, an alkyl group, or an acyl group, more preferably a hydrogen atom or an acyl group, and further preferably a hydrogen atom or an acetyl group.

An isotope of a fluorine atom for X^{L1}, X^{L3}, X^{L4}, X^{L5}, X^{D1}, X^{D3}, X^{D4}, or X^{D5} is not particularly limited. Examples thereof include a radioactive isotope ¹⁸F, a stable isotope ¹⁹F, or the like. When the isotope of a fluorine atom is ¹⁸F, the glucose derivative according to the present embodiment may be suitably used as, for example, a PET reagent. When the isotope of a fluorine atom is ¹⁹F, the glucose derivative according to the present embodiment may be suitably used as, for example, a cancer drug. From the viewpoint of making the above-mentioned PET reagent or cancer drug application more suitable, for General Formula (1), X^{L4} or X^{L5} is preferably a fluorine atom and, for General Formula (2), X^{D4} or X^{D5} is preferably a fluorine atom.

From the viewpoint of easiness of synthesis or activity of the glucose derivative, X^{L1}, X^{L3}, X^{L4}, and X^{L5} each independently preferably is an -OR^{L1} group, and X^{D1}, X^{D3}, X^{D4}, and X^{D5} each independently preferably is -OR^{D1} group.

The L-glucose derivative represented by General Formula (1) may be synthesized from, for example, an L-form sugar or a derivative thereof. The D-glucose derivative represented by General Formula (2) may be synthesized from, for example, a D-form sugar or a derivative thereof. Specific examples of the L-form sugar or a derivative thereof include L-glucose, L-mannose, L-galactose, 1,2:5,6-di-O-isopropylidene-α-L-glucofuranose, or the like. Specific examples of the D-form sugar or a derivative thereof include D-glucose, D-mannose, D-galactose, 1,2:5,6-di-O-isopropylidene-α-D-glucofuranose, or the like.

A method for synthesizing the L-glucose derivative represented by General Formula (1) from the L-form sugar or a derivative thereof and a method for synthesizing the D-glucose derivative represented by General Formula (2) from the D-form sugar or a derivative thereof may be, for example, methods described in Examples, or the like. Various derivatives can be synthesized by combining steps of the methods described in Examples as appropriate or by introducing substituents using conventionally known methods.

A method other than the methods described in Examples is not particularly limited, but, for example, the below-described methods may be used.

A glucose derivative having a structure represented by General Formula (1) in which X^{L2} denotes an -SAuP(C₂H₅)₃ group, X^{L1}, X^{L3}, and X^{L4} denote an -OH group, and X^{L5} denotes a fluorine atom can be synthesized as Compound 11 using L-mannose as a starting material according to the below-described reaction formula. In Compound 11, an OH group at position 6 of glucose is substituted with a fluorine atom.

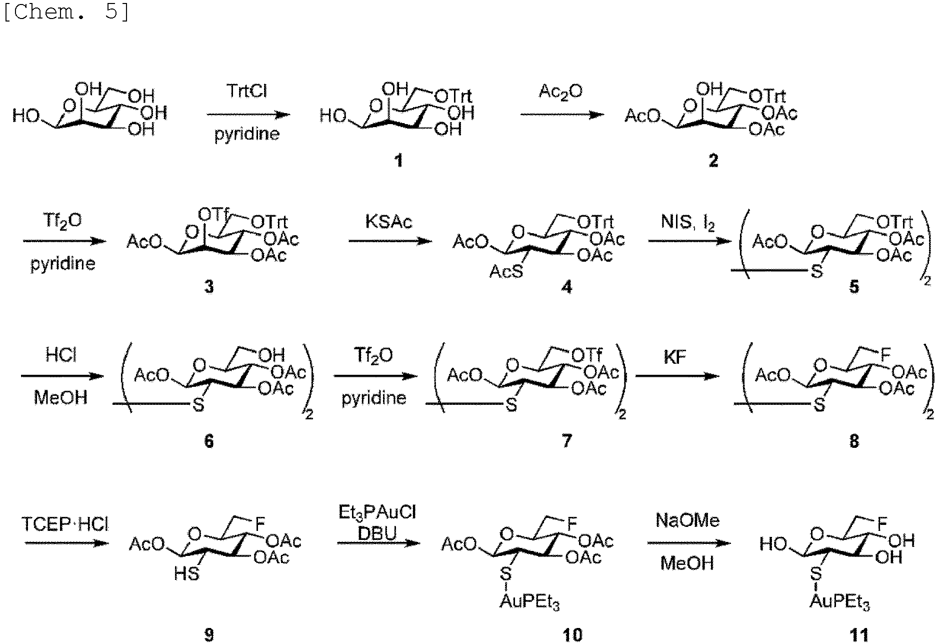

A glucose derivative having a structure represented by General Formula (1) in which X^{L2} denotes an -SAuP(CH₃)₃ group, X^{L1}, X^{L3}, and X^{L4} denote an -OH group, and X^{L5} denotes a fluorine atom can be synthesized according to the above-described reaction formula, except that Et is replaced by Me in the reactions from Compound 9 to Compound 11.

A glucose derivative having a structure represented by General Formula (1) in which X^{L2} denotes an -SAuP(C₂H₅)₃ group, X^{L1}, X^{L3}, and X^{L5} denote an -OH group, and X^{L4} denotes a fluorine atom can be synthesized as Compound 20 using Compound 12 as a starting material according to the below-described reaction formula. In Compound 20, an OH group at position 4 of glucose is substituted with a fluorine atom. Note that, Compound 12 is Compound 5L in the below-described Examples.

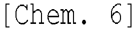

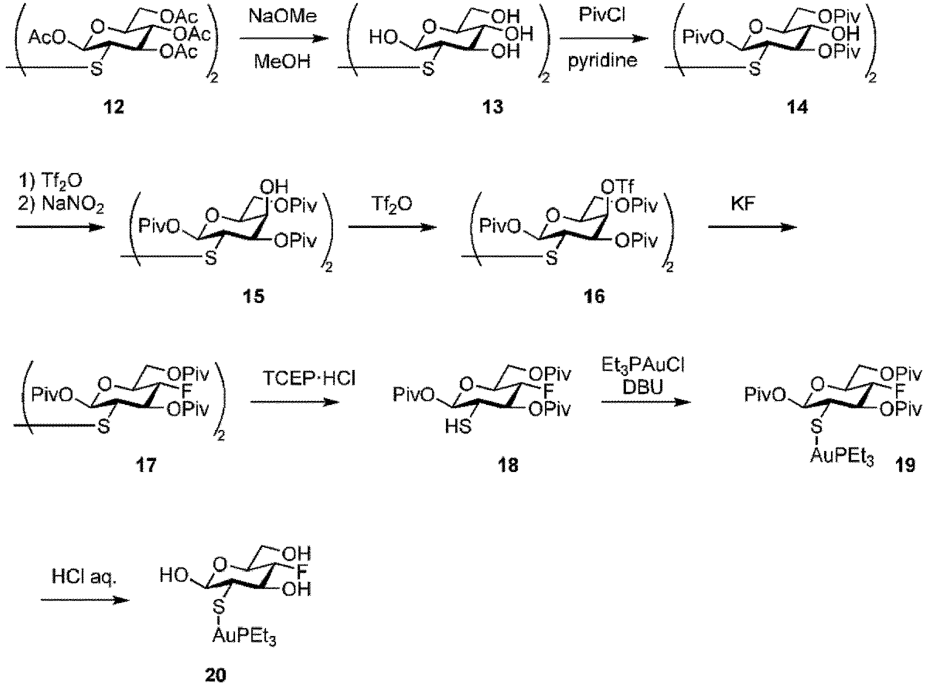

A glucose derivative having a structure represented by General Formula (1) in which X^{L2} denotes an -SAuP(CH₃)₃ group, X^{L1}, X^{L3}, and X^{L5} denote an -OH group, and X^{L4} denotes a fluorine atom can be synthesized according to the above-described reaction formula, except that Et is replaced by Me in the reactions from Compound 18 to Compound 20.

The glucose derivative according to the present embodiment can be used to effectively treat various cancers, in particular, adenocarcinomas such as pancreatic cancer, brain tumor, breast cancer, gastric cancer, gastroesophageal junction cancer, oral cancer, biliary tract cancer, lung cancer, ovarian cancer, and uterine cancer; and sarcoma. Therefore, the glucose derivative according to the present embodiment can be suitably used as an anticancer agent.

An anticancer agent according to the present embodiment comprising at least one of the glucose derivatives according to the present embodiment. Cancer for which the above-described anticancer agent is applied is not particularly limited. Examples thereof include various cancers such as adenocarcinomas such as pancreatic cancer, brain tumor, breast cancer, gastric cancer, gastroesophageal junction cancer, oral cancer, biliary tract cancer, lung cancer, ovarian cancer, and uterine cancer; and sarcoma.

The glucose derivative according to the present embodiment or the anticancer agent according to the present embodiment can be administered, for example, in the form of an oral agent (a tablet, a granule, a powder, a capsule, a syrup, etc.), a sublingual tablet, or an injection (for intravenous administration, intramuscular administration, subcutaneous administration, intraperitoneal administration, intraarticular administration, epidural administration, etc.), or in the form of direct application to the cervix, the oral cavity, the skin, etc.

### EXAMPLES

Hereinafter, the present invention will be further specifically described with reference to Examples, but the present invention is not limited to Examples in any way. Note that, structures of compounds synthesized were identified using UPLC-MS, ¹H-NMR, ¹³C-NMR, or the like. A sample for specific optical rotation was measured for specific optical rotation five times at 20.00°C using a cell with a cell length of 100 mm and an average value was calculated from the five measurement results. This average value was used as a value of specific optical rotation in each example.

Four types of 2-thio-glucose derivatives to which trialkylphosphine gold was attached were synthesized. The four types of compounds are as follows: a first compound is Compound A (M2-ADG) of which sugar moiety is D-glucose and in which trimethylphosphine gold is attached to a thiol group at position 2; a second compound is Compound B (E2-ADG) of which sugar moiety is D-glucose and in which triethylphosphine gold is attached to a thiol group at position 2; a third compound is Compound C (M2-ALG) of which sugar moiety is L-glucose and in which trimethylphosphine gold is attached to a thiol group at position 2; and a fourth compound is Compound D (E2-ALG) of which sugar moiety is L-glucose and in which triethylphosphine gold is attached to a thiol group at position 2.

### [Example 1]

### · Compound A (M2-ADG)

Compound A (M2-ADG) was synthesized using D-mannose (Compound 1D) as a starting material according to the following reaction formula:

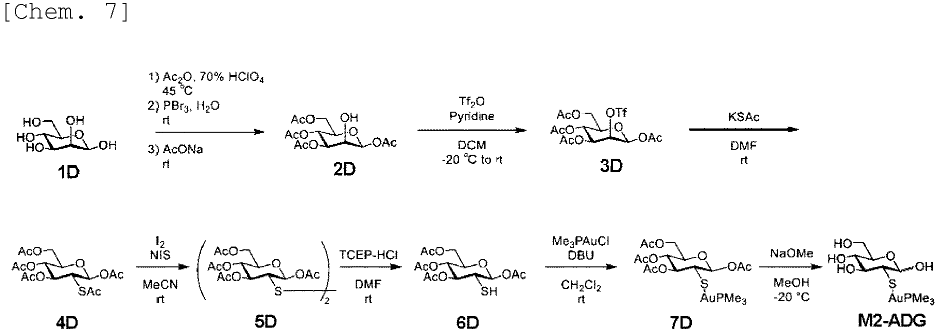

### · Synthesis of Compound 1D to Compound 4D

Compound 4D was obtained according to the formulation reported in the article (J. Am. Chem. Soc. 2010, 132, 7405-7417).

### · Synthesis of Compound 6D

For a process of obtaining Compound 6D from Compound 4D via Compound 5D, Compound 6D was obtained by forming a disulfide dimer Compound 5D via an oxidization reaction followed by reduction to selectively remove a S-acetyl group with reference to the article (J. Org. Chem. 2017, 82, 12613-12623).

### · Synthesis of Compound 2D

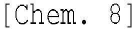

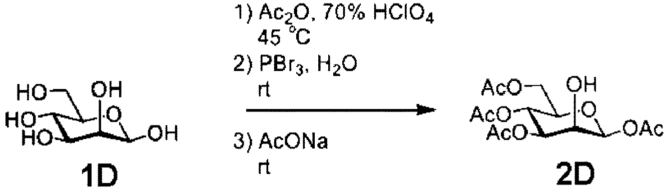

Acetic anhydride (95 mL, 1.0 mol), 50 mg of D-mannose (Compound 1D), and 70% by mass of HClO₄ (0.50 mL) were added to a 1 L flask and heated to 40°C. Then, the remaining D-mannose (24.95 g, 138.5 mmol) was charged in portions at the same temperature and stirred at room temperature overnight. Then, the reaction vessel was cooled in an ice bath, PBr₃ (19.8 mL, 139 mmol) was added dropwise thereto, and then cold water (13.6 mL) was added dropwise thereto at 27°C or less. After completion of the addition, the resulting reaction solution was brought to room temperature and stirred for 1 hour. The resultant was cooled in an ice bath again and a solution of sodium acetate (45.6 g, 556 mmol) in water (57 mL) was added dropwise thereto. After stirring at room temperature for 1 hour, the resulting reaction solution was poured into 83 mL of cold water and extracted twice with dichloromethane. The resultant was then washed twice with cold saturated sodium bicarbonate water, reextracted with dichloromethane, and dried over Na₂SO₄. After filtering out a drying agent and concentrating under reduced pressure, diethyl ether (225 mL) was added to the resulting crude containing Compound 2D to deposit a white solid. The thus-deposited solid was filtered out and washed with cold diethyl ether to give 8.4 g (yield: 17%) of Compound 2D.

### · Synthesis of Compound 4D

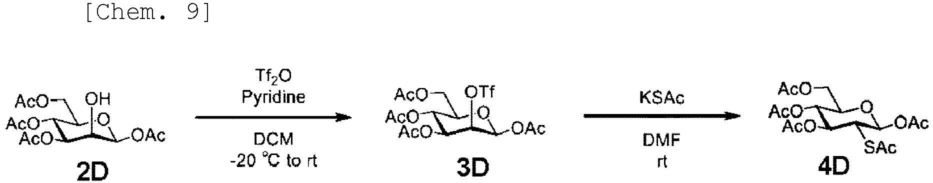

Compound 2D (8.4 g, 24.1 mmol), dehydrated dichloromethane (252 mL), and dehydrated pyridine (4.3 mL, 53.1 mmol) were added in a 500 mL flask under an argon atmosphere and cooled so that an internal temperature reached -20°C. Then, Tf₂O (8.7 mL, 53.1 mmol) was added so that an internal temperature did not exceed -20°C and stirred at the same temperature. After completion of the reaction, the resulting reaction solution was poured into cold water, washed with cold saturated sodium bicarbonate water (340 mL), dried over Na₂SO₄, filtered, and concentrated to give crude Compound 3D as a brown oil. The resultant was used in the subsequent step without further purification.

Compound 3D (11.6 g, 24.1 mmol), DMF (440 mL), and KSAc (27.6 g, 241 mmol) were added in a 1 L flask under an argon atmosphere and stirred at room temperature. After completion of the reaction, the resultant was extracted with water/dichloromethane (volume ratio: 1/3), washed with brine, and concentrated under reduced pressure. The resulting residue containing Compound 4D was purified with silica gel (216 g). Ethyl acetate/heptane (volume ratio: 1/3) was used as an eluent. A fraction containing a substance of interest was concentrated to obtain 9.4 g of crude Compound 4D. The crude was recrystallized with ethyl acetate/heptane (volume ratio: 1/2) to obtain 5.8 g (yield: 66%) of Compound 4D.

### · Synthesis of Compound 5D

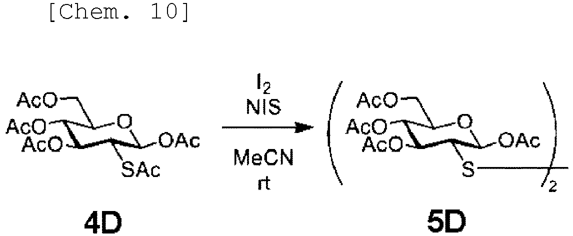

This reaction was carried out in four separate batches. Under an argon atmosphere, Compound 4D (1.0 g, 2.5 mmol), dehydrated acetonitrile (15 mL), iodine (1.6 g, 6.2 mmol), and NIS (1.4 g, 6.2 mmol) were added and stirred for 5 hours at room temperature. (The same operation was performed in three more batches using 1.0 g of Compound 4D.) The resulting reaction solution was then poured into water/dichloromethane (volume ratio: 1/3), and the resulting organic layer was washed with a 5% by mass sodium thiosulfate aqueous solution and brine, dried over Na₂SO₄, filtered, and concentrated under reduced pressure. The resulting residue containing Compound 5D was purified with silica gel (106 g). Ethyl acetate/heptane (volume ratio: 1/2) was used as an eluent. A fraction containing a substance of interest was concentrated to obtain 1.7 g of crude Compound 5D. Diisopropyl ether IPE (34 mL) was then added to the resulting crude at room temperature to deposit a solid. The thus-deposited solid was filtered out and dried to give 1.5 g (yield: 41%) of Compound 5D.

### · Synthesis of Compound 7D

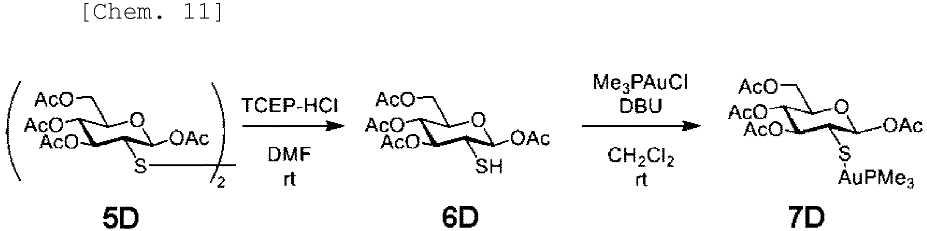

Compound 5D (371 mg, 511 µmol), DMF (3.7 mL), and TCEP·HCl (192 mg, 766 µmol) were added in a 20 mL eggplant flask under an argon atmosphere. After stirring at room temperature for 1 hour, the resultant was extracted using water/dichloromethane (volume ratio: 1/3), washed with brine, dried over Na₂SO₄, filtered, and concentrated under reduced pressure. The resultant was azeotroped with toluene to obtain crude Compound 6D. The resultant was used in the subsequent step without further purification.

Compound 6D, dehydrated dichloromethane (3.7 mL), and Me₃PAuCl (283 mg, 916 µmol) were added in a 20 mL eggplant flask under an argon atmosphere. DBU (182 µL, 1.2 mmol) was then dissolved in dehydrated dichloromethane (927 µL) and added dropwise to the resulting reaction solution. After completion of the reaction was confirmed, the resultant was filtered through Celite and concentrated under reduced pressure. The resulting residue containing Compound 7D was purified with NH silica gel (20 g). Ethyl acetate/heptane (volume ratio: 1/1) was used as an eluent. A fraction containing a substance of interest was concentrated to obtain 367 mg (yield: 56%) of Compound 7D.

### · Synthesis of Compound A (M2-ADG)

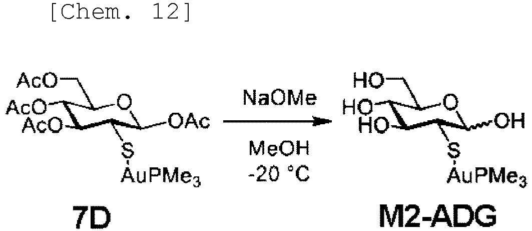

Compound 7D (360 mg, 566 µmol) and methanol (5.5 mL) were added in a 20 mL eggplant flask under an argon atmosphere and cooled so that an internal temperature reached -20°C. Then, a 5 M NaOMe solution in methanol (136 µL, 679 µmol) was added dropwise thereto and stirred at the same temperature. After completion of the reaction was confirmed, Amberlite IR120 (H⁺ form) was added to bring the pH to 6 to 7, followed by filtration, and then concentration under reduced pressure. The resulting residue containing M2-ADG was purified with DIOL silica gel (15 g). Chloroform/methanol (volume ratio: 100/0 to 98/2 to 96/4) was used as an eluent. A fraction containing a substance of interest was concentrated and lyophilized to give 127 mg (yield: 47%) of M2-ADG.

### · Measurement of specific optical rotation

Compound A (M2-ADG) was dissolved in water to a concentration of 0.9408 (g/dL) to obtain a sample for specific optical rotation measurement. The specific optical rotation was determined as +30.41 degrees.

### [Example 2]

### · Compound B (E2-ADG)

Compound B (E2-ADG) was synthesized using Compound 5D of Example 1 as a starting material according to the following reaction formula:

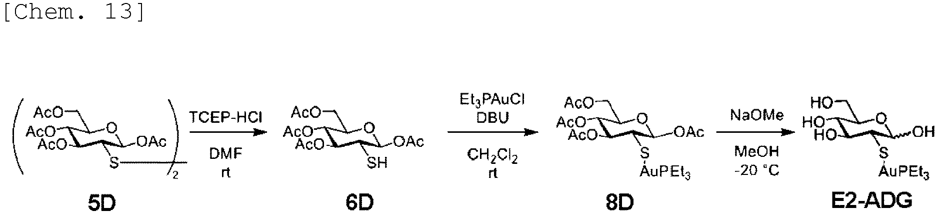

### · Synthesis of Compound 8D

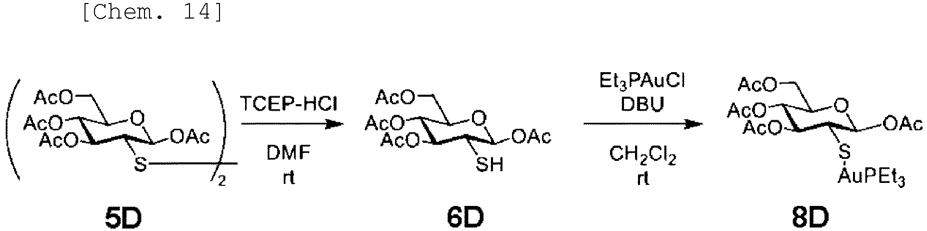

Compound 5D (1.0 g, 1.4 mmol), DMF (10.4 mL), and TCEP·HCl (540 mg, 2.1 mmol) were added in a 30 mL eggplant flask under an argon atmosphere. After stirring at room temperature for 1 hour, the resultant was extracted using water/dichloromethane (volume ratio: 1/3), washed with brine, dried over Na₂SO₄, filtered, and concentrated under reduced pressure. The resultant was azeotroped with toluene to obtain crude Compound 6D. The resultant was used in the subsequent step without further purification.

Compound 6D, dehydrated dichloromethane (10.4 mL), and Et₃PAuCl (900 mg, 2.6 mmol) were added in a 50 mL flask under an argon atmosphere. DBU (512 µL, 3.4 mmol) was then dissolved in dehydrated dichloromethane (2.6 mL) and added dropwise to the resulting reaction solution. After completion of the reaction was confirmed, the resultant was concentrated under reduced pressure. The resulting residue containing Compound 8D was purified with NH silica gel (62 g). Ethyl acetate/heptane (volume ratio: 1/1) was used as an eluent. A fraction containing a substance of interest was concentrated to obtain 1.6 g (yield: 85%) of Compound 8D.

### · Synthesis of Compound B (E2-ADG)

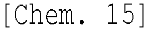

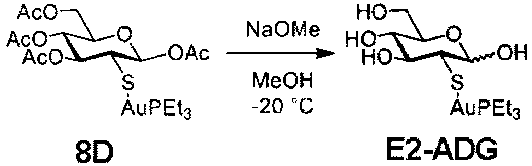

Compound 8D (1.63 g, 2.4 mmol) and methanol (24 mL) were added in a 100 mL flask under an argon atmosphere and cooled so that an internal temperature reached -20°C. Then, a 5 M NaOMe solution in methanol (577 µL, 2.8 mmol) was added dropwise thereto and stirred at the same temperature. After completion of the reaction was confirmed, Amberlite IR120 (H⁺ form) was added to bring the pH to 6 to 7, followed by filtration, and then concentration under reduced pressure. The resulting residue containing E2-ADG was purified with DIOL silica gel (15 g). Chloroform/methanol (volume ratio: 100/0 to 98/2 to 96/4) was used as an eluent. A fraction containing a substance of interest was concentrated and lyophilized to give 136 mg (yield: 11%) of E2-ADG.

### · Measurement of specific optical rotation

Compound B (E2-ADG) was dissolved in water to a concentration of 0.9410 (g/dL) to obtain a sample for specific optical rotation measurement. The specific optical rotation was determined as +34.01 degrees.

### [Example 3]

### · Compound C (M2-ALG)

Compound C (M2-ALG) was synthesized using L-mannose (Compound 1L) as a starting material according to the following reaction formula:

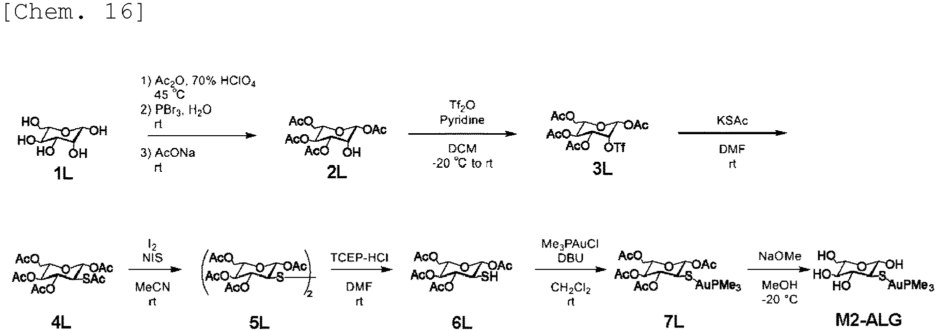

### · Synthesis of Compound 2L

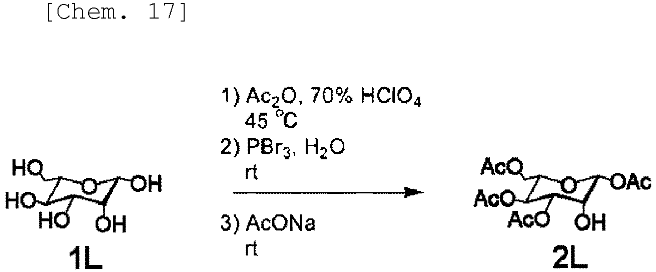

Acetic anhydride (62 g, 0.60 mol), 50 mg of L-mannose (Compound 1L), and 70% by mass of HClO₄ (0.30 mL) were added to a 1 L flask and heated to 40°C. Then, the remaining L-mannose(14.95 g, 83 mmol) was charged in portions at the same temperature and stirred at room temperature for 17 hours. Then, the reaction vessel was cooled in an ice bath, PBr₃ (22.5 g, 83 mmol) was added dropwise thereto, and then cold water (13.6 mL) was added dropwise thereto at 27°C or less. After completion of the addition, the resulting reaction solution was brought to room temperature and stirred for 1.5 hours. After progress of the reaction was confirmed by TLC, the resultant was cooled again in an ice bath and a solution of sodium acetate (27.3 g, 333 mmol) in water (34 mL) was added dropwise thereto. After stirring at room temperature for 20 minutes, the resulting reaction solution was poured into 50 mL of an ice bath and extracted twice with dichloromethane. The resultant was then washed twice with cold saturated sodium bicarbonate water, reextracted with dichloromethane, and dried over Na₂SO₄. After filtering out a drying agent and concentrating under reduced pressure, 150 mL of diethyl ether was added to deposit a white solid. The thus-deposited solid was filtered out and washed with cold diethyl ether to give 6.5 g (yield: 23%) of Compound 2L.

### · Synthesis of Compound 4L

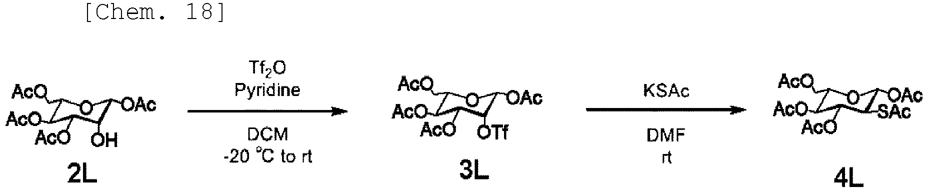

Compound 2L (18.8 g, 2.6 mmol), dehydrated pyridine (3.33 mL, 41.2 mmol), and dehydrated dichloromethane (196 mL) were added in a 500 mL flask under an argon atmosphere and cooled so that an internal temperature reached -20°C. Then, Tf₂O (6.76 mL, 41.2 mmol) was added so that an internal temperature did not exceed -20°C and stirred at the same temperature. After completion of the reaction, the resulting reaction solution was poured into cold water, washed with cold saturated sodium bicarbonate water (150 mL), dried over Na₂SO₄, filtered, and concentrated to give 10.1 g of crude Compound 3L as a brown oil. The resultant was used in the subsequent step without further purification.

Compound 3L (9.0 g, 18.7 mmol), DMF (342 mL), and KSAc (21.4 g, 187 mmol) were added in a 1 L flask under an argon atmosphere and stirred at room temperature. After completion of the reaction, the resultant was extracted with water/ethyl acetate (volume ratio: 1/3), washed with brine, and concentrated under reduced pressure. The resulting residue containing Compound 4L was purified with silica gel (272 g). Ethyl acetate/heptane (volume ratio: 1/3) was used as an eluent. A fraction containing a substance of interest was concentrated to obtain 7.8 g of crude Compound 4L. The crude was recrystallized with ethyl acetate/heptane (volume ratio: 1/2) to give 5.0 g (yield: 66%) of Compound 4L.

### · Synthesis of Compound 5L

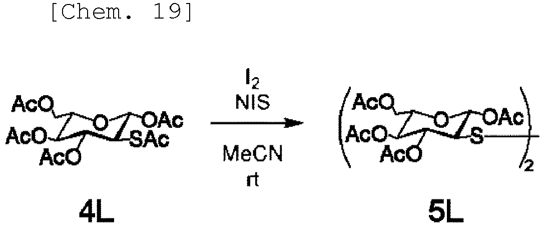

This reaction was carried out in two separate batches. Under an argon atmosphere, Compound 4L (1.5 g, 3.7 mmol), dehydrated acetonitrile (30 mL), iodine (2.3 g, 9.2 mmol), and NIS (2.1 g, 9.2 mmol) were added and stirred for 6 hours at room temperature. (The same operation was performed in one more batch using 1.5 g of Compound 4L.) The resulting reaction solution was then poured into water/dichloromethane (volume ratio: 1/3), and the resulting organic layer was washed with a 5% by mass sodium thiosulfate aqueous solution and brine, dried over Na₂SO₄, filtered, and concentrated under reduced pressure. The resulting residue containing Compound 5L was purified with silica gel (55 g). Ethyl acetate/heptane (volume ratio: 1/1) was used as an eluent. A fraction containing a substance of interest was concentrated to obtain 1.5 g of crude Compound 5L. Diisopropyl ether IPE (15 mL) was then added to the resulting crude at room temperature to deposit a solid. The thus-deposited solid was filtered out and dried to give 993 mg (yield: 310) of Compound 5L.

### · Synthesis of Compound 7L

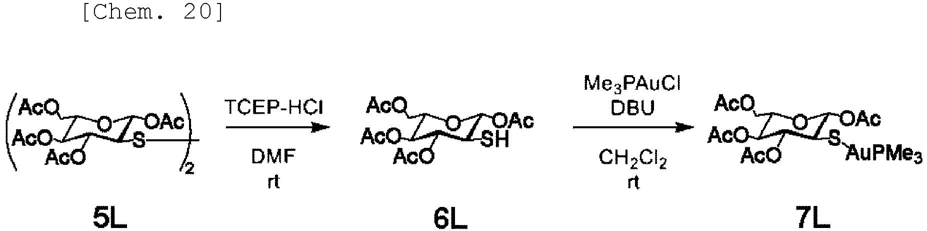

Compound 5L (335 mg, 0.461 mmol), DMF (3.3 mL), and TCEP·HCl (198 mg, 0.690 mmol) were added in a 30 mL flask under an argon atmosphere. After stirring at room temperature for 2 hours, the resultant was extracted using water/dichloromethane (volume ratio: 1/3), washed with brine, dried over Na₂SO₄, filtered, and concentrated under reduced pressure. Furthermore, the resultant was azeotroped with toluene to obtain crude Compound 6L (light yellow oil). The resultant was used in the subsequent step without further purification.

Compound 6L, dehydrated dichloromethane (3.4 mL), and Me₃PAuCl (281 mg, 0.910 mmol) were added in a 10 mL flask under an argon atmosphere. DBU (168 mg, 1.10 mmol) was then dissolved in dehydrated dichloromethane (0.84 mL) and added dropwise to the resulting reaction solution. After completion of the reaction was confirmed, the resultant was filtered through Celite and concentrated under reduced pressure. The resulting residue containing Compound 7L was purified with NH silica gel (1.8 g). Ethyl acetate/heptane (volume ratio: 1/1) was used as an eluent. A fraction containing a substance of interest was concentrated to obtain 430 mg (yield: 73%) of Compound 7L.

### · Synthesis of Compound M2-ALG

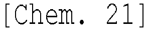

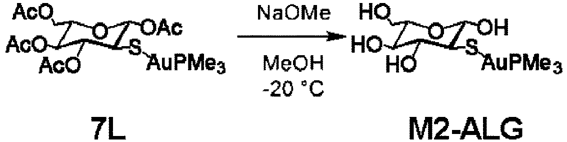

Compound 7L (373 mg, 0.59 mmol) and methanol (5.6 mL) were added in a 10 mL flask under an argon atmosphere and cooled so that an internal temperature reached -20°C. Then, a 5 M NaOMe solution in methanol (0.14 mL, 0.70 mmol) was added dropwise and stirred at the same temperature. After completion of the reaction was confirmed, Amberlite IR120 (H⁺ form) was added to bring the pH to 6 to 7, followed by filtration, and then concentration under reduced pressure. The resulting residue containing M2-ALG was purified with DIOL silica gel (10 g). Chloroform/methanol (volume ratio: 100/0 to 98/2 to 96/4) was used as an eluent. A fraction containing a substance of interest was concentrated and lyophilized to give 136 mg (yield: 50%) of M2-ALG.

### · Measurement of specific optical rotation

Compound C (M2-ALG) was dissolved in water to a concentration of 0.6224 (g/dL) to obtain a sample for specific optical rotation measurement. The specific optical rotation was determined as -31.65 degrees.

### [Example 4]

### · Compound D (E2-ALG)

Compound D (E2-ALG) was synthesized using Compound 5L of Example 3 as a starting material according to the following reaction formula:

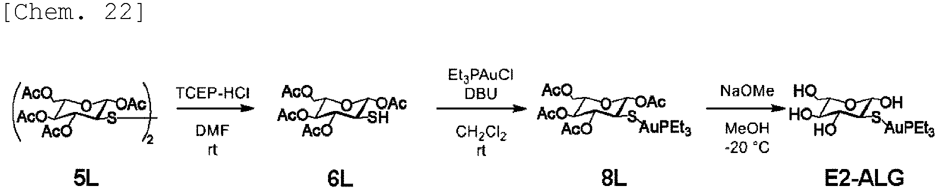

### · Synthesis of Compound 8L

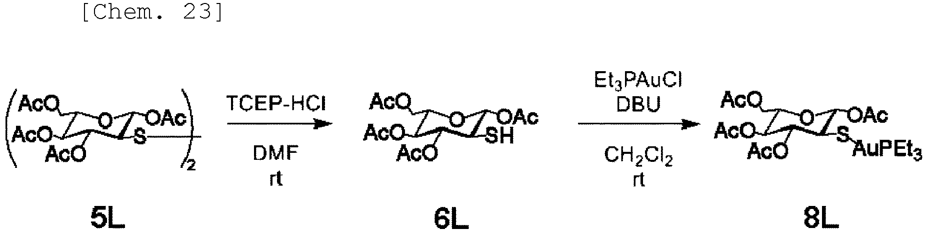

Compound 5L (444 mg, 0.61 mmol), DMF (4.4 mL), and TCEP·HCl (263 mg, 0.92 mmol) were added in a 30 mL flask under an argon atmosphere. After stirring at room temperature for 2 hours, the resultant was extracted using water/dichloromethane (volume ratio: 1/3), washed with brine, dried over Na₂SO₄, filtered, and concentrated under reduced pressure. The resultant was azeotroped with toluene to obtain crude Compound 6L. The resultant was used in the subsequent step without further purification.

Compound 6L, dehydrated dichloromethane (4.4 mL), and Et₃PAuCl (423 mg, 1.21 mmol) were added in a 100 mL eggplant flask under an argon atmosphere. DBU (223 mg, 1.5 mmol) was then dissolved in dehydrated dichloromethane (1.0 mL) and added dropwise to the resulting reaction solution. After completion of the reaction was confirmed, the resultant was concentrated under reduced pressure. The resulting residue containing Compound 8L was purified with NH silica gel (24.4 g). Ethyl acetate/heptane (volume ratio: 1/1) was used as an eluent. A fraction containing a substance of interest was concentrated to obtain 541 mg (yield: 65%) of Compound 8L.

### · Synthesis of Compound E2-ALG

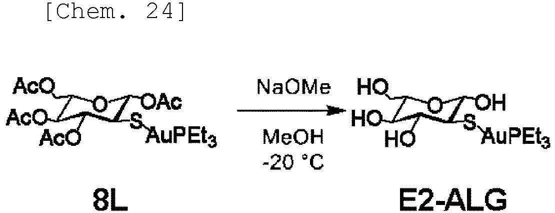

Compound 8L (541 mg, 0.797 mmol) and methanol (8.1 mL) were added in a 30 mL flask under an argon atmosphere and cooled so that an internal temperature reached -20°C. Then, a 5 M NaOMe solution in methanol (0.19 mL, 0.96 mmol) was added dropwise and stirred at the same temperature. After completion of the reaction was confirmed, Amberlite IR120 (H⁺ form) was added to bring the pH to 6 to 7, followed by filtration, and then concentration under reduced pressure. The resulting residue containing E2-ALG was purified with DIOL silica gel (10 g). Chloroform/methanol (volume ratio: 100/0 to 98/2 to 96/4) was used as an eluent. A fraction containing a substance of interest was concentrated and lyophilized to give 185 mg (yield: 46%) of E2-ALG.

### · Measurement of specific optical rotation

Compound D (E2-ALG) was dissolved in water to a concentration of 0.6430 (g/dL) to obtain a sample for specific optical rotation measurement. The specific optical rotation was determined as -32.12 degrees.

### <Administration test using E2-ADG or E2-ALG>

### [Example 5-1] Administration to MIN6 cells early after culture initiation

Effects of E2-ADG (synthesized in Example 2) and E2-ALG (synthesized in Example 4) on proliferation of cells were examined. A pancreatic beta cell line (insulinoma) MIN6 from mouse pancreas (Miyazaki J. et al., Endocrinology 127: 126-132, 1990) was used as the cells, and all experiments were performed using early passage cells with a passage number of 10 or less.

MIN6 cells are one of highly reliable cells widely used in the world as a normal pancreatic beta cell line in the islets of Langerhans secreting insulin (Miyazaki J. et al., Endocrinology 127: 126-132, 1990), and, from initiation of culture to approximately 6 DIV, fluorescent D-glucose 2-NBDG is taken up by the cells mainly via a glucose transporter GLUT (Yamada, K. et al., J. Biol. Chem. 275: 22278-83, 2000; Yamada, K. Nature Protocols 2: 753-62, 2007; and Sasaki, A. et al., Human Cell 2016, 29, 37-45).

Previous studies have shown that application of fluorescent D-glucose 2-NBDG to MIN6 cells with a passage number of 10 or less at less than 6 DIV of culture results in GLUT-mediated uptake, whereas uptake of fluorescent L-glucose 2-NBDLG is only occasional and slight, and very weak on the whole; after 7 DIV of culture, a small fraction of the MIN6 cells clearly show intracellular uptake of the 2-NBDLG; and after 9 DIV of culture, uptake of the 2-NBDLG into the MIN6 cells became evident, with the maximal uptake of the 2-NBDLG being found at 10 DIV to 15 DIV (Sasaki, A. et al., Human Cell 2016, 29, 37-45). In addition, it has been previously reported that the uptake of the 2-NBDLG into the MIN6 cells at 10 DIV to 15 DIV is not mediated by a glucose transporter such as GLUT, but by a "channel-like transport mechanism that does not distinguish between D-glucose and L-glucose (Sasaki, A. et al., Human Cell 2016, 29, 37-45); and that this "channel-like transport mechanism that does not distinguish between D-glucose and L-glucose" is selectively inhibited by Phloretin at 150 µM, and the uptake into the MIN6 cells is almost eliminated in the presence of Phloretin at this concentration (Sasaki, A. et al., Human Cell 2016, 29, 37-45).

Based on the above findings, the present inventors expected that, if E2-ALG does not pass through the GLUT but is taken up into MIN6 cells via the above- described channel-like transport mechanism as was the case with application of the 2-NBDLG, then the E2-ALG would be hardly taken up into the MIN6 cells from initiation of culture to 6 DIV, and an effect of the E2-ALG on cell proliferation would be minimal.

In this experiment, approximately 3,000 MIN6 cells per well were seeded in each well of a 96-well plate. When 5 DIV elapsed from initiation of culture, wells to which E2-ALG was administrated and wells to which E2-ADG was administrated were arranged in the same plate with minimal external disturbance, and administration was performed according to the previously established method (Sasaki, A. et al., Human Cell 2016, 29, 37-45). The E2-ALG was dissolved in a buffer to a concentration of 1,000 µM, administered to the MIN6 cells for 10 or 30 minutes, and then washed out with a buffer solution without the E2-ALG. Meanwhile, the E2-ADG at a concentration of 1,000 µM was administered in the same manner. Carbenoxolone was pre-dissolved in a buffer solution for the purpose of excluding uptake via Hemichannel and Gap junction (Sasaki, A. et al., Human Cell 2016, 29, 37-45).

A change in cellular activity was assessed using a live cell marker Calcein-AM. The Calcein-AM is permeable to a cell membrane, and after entering a cell, an AM moiety is cleaved by an esterase activity of the cell to produce Calcein which emits green fluorescence. The Calcein-AM was applied to the MIN6 cells according to a conventional method, green fluorescence emitted by the cells was quantitatively measured using a confocal quantitative image cytometer CellVoyager CQ1 (Yokogawa Electric Corporation), and image analysis was performed using software attached to the CQ1. A difference between an E2-ALG administration group and an E2-ADG administration group was statistically compared using Stat View 5.0. The Bonferroni-Dunn test was used for the statistical comparison. A value shown by MIN6 cells at the same time as above in a Krebs-Ringer buffer (KRB) solution without E2-ALG or E2-ADG was used as a negative control.

An extent of occurrence of cell death was assessed using Propidium Iodide (PI), a red dye that is impermeable to a cell membrane. The PI was applied to the MIN6 cells simultaneously with the Calcein-AM. The principle of assessing cell death using PI is as follows. The PI does not enter a cell with a healthy cell membrane and fluorescence is not emitted. However, when the PI nonspecifically enters a cell due to loss of integrity of a cell membrane caused by deterioration of a cell state, the PI binds to a nucleus of the cell, causing the nucleus to emit red fluorescence. A degree of the red fluorescence was quantitatively measured using the CellVoyager CQ1, and image analysis was performed using the software attached to the CQ1. Additionally, the degree of occurrence of cell death was evaluated by statistically comparing the control group with the administration groups or the E2-ALG administration group with the E2-ADG administration group.

First, an effect of the E2-ALG on the MIN6 cells at 5 DIV was examined using green fluorescence of Calcein which indicates a cellular activity. Results of the experiment showed that 10-minute administration of the E2-ALG slightly decreased an average value of the cellular activity compared to the negative control group, but there was no statistically significant difference between the negative control group and the E2-ALG administration group (Fig. 1). When the E2-ALG was administered for 30 minutes, the cellular activity was clearly decreased compared to the negative control, and this decrease was statistically significant (Fig. 1).

Assessment of occurrence of cell death using a dead cell marker Propidium Iodide (PI) simultaneously performed showed that the administration groups had an average value higher than that for the negative control KRB, but measurement values varied widely, with no statistically significant difference between the negative control group and E2-ALG, between the negative control and the E2-ADG, and between the E2-ALG and the E2-ADG for both 10-minute and 30-minute administrations (not shown).

However, when cytotoxicity of the E2-ADG administered to the MIN6 cells at 5 DIV for 10 minutes was compared to that of the E2-ALG administered for 10 minutes as well, contrary to our expectation, an average value of the cellular activity expressed as Calcein intensity for the E2-ADG was slightly lower than that for the E2-ALG, but there was no statistically significant difference therebetween (Fig. 1). Similar results were obtained for the 30-minute administration (Fig. 1). These results were completely unexpected.

In fact, the above-described experimental results are completely different from that, when a D-glucose analog DGG and an L-glucose analog LGG in which a hydrogen atom in an OH group at position 1 was substituted with the same -SAuP(C₂H₅)₃ group as in the present invention instead of a hydrogen atom in an OH group at position 2 of glucose (Yamaguchi E. et al., PCT/JP2021/013857) were administered to the same MIN6 cells as in the present invention at 5 DIV at a concentration of 1,000 µM for 30 minutes, the DGG caused distinct PI-positive cell death but the LGG showed only about as much PI-positive cell death as a negative control group (Fig. 2). Similarly, when administrated at 1,000 µM for 10 minutes, the DGG caused more PI-positive cell death than the negative control, whereas the LGG showed PI-positive cell death comparable to that of the negative control (Fig. 2).

Results for administration of the DGG or the LGG show that the DGG enters MIN6 cells at a time when few cells exhibit a malignant tumor cell feature such as dyskaryosis through a protein that selectively interacts with D-glucose such as a glucose transporter and exhibits cytotoxicity to the MIN6 cells but the LGG which cannot pass through the glucose transporter does not exhibit cytotoxicity to these cells under the same conditions. These results are distinctly different from the results obtained in the present invention that not only the E2-ADG but also the E2-ALG administered to the same MIN6 cells at 5 DIV at the same concentrations and for the same durations as described above caused a clear decrease in cellular activity and there was no statistically significant difference between the D and L forms.

These results when the E2-ADG or the E2-ALG was administered to the MIN6 cells at 5 DIV at a concentration of 1,000 µM for 10 or 30 minutes may be explained by assuming that "not only the E2-ALG but also the E2-ADG cannot pass through the glucose transporter GLUT expressed in the MIN6 cells at 5 DIV " but both the E2-ALG and the E2-ADG intensely enter the cell via the "channel-like transport mechanism that does not distinguish between D-glucose and L-glucose" which is slightly expressed in the MIN6 cells at 5 DIV and distinctly reduce an activity of the cells to which the E2-ADG or the E2-ALG has entered, albeit not consistently causing cell death as assessed in PI-positive cells. Therefore, the below-described experiments were further conducted to validate this

### hypothesis.

### [Example 5-2] Administration to MIN6 cells after 10 DIV from initiation of culture

Using MIN6 cells with a passage number of 10 or less, E2-ALG or E2-ADG was applied to the MIN6 cells at a concentration of 1,000 µM for 90 minutes at 11 DIV of culture at a time when spheroids with a diameter of 100 um or more are normally formed. Specifically, approximately 1,000 MIN6 cells per well were seeded in each well of a 96-well plate and E2-ALG was dissolved in a buffer to a concentration of 10 µM or 1,000 µM at 11 DIV, administered to the MIN6 cells for 90 minutes, and then rapidly washed out with a buffer solution without E2-ALG. Similarly, E2-ADG was applied simultaneously to other wells in the same plate. A cell death marker PI was used to statistically compare a control group with administration groups or an E2-ALG administration group with an E2-ADG administration group (Fig. 3).

Results of the experiment showed that both the E2-ALG and the E2-ADG caused a significant increase in a proportion of dead cells expressed as a PI value and thus strong cytotoxicity in the MIN6 cells at 11 DIV compared to the negative control, that is, a KRB solution alone to which neither E2-ALG nor E2-ADG was added (Fig. 3). A degree of an effect of the E2-ALG on the cells was slightly lower than that of the E2-ADG as an average value, but there was no statistically significant difference between them (Fig. 3). At a concentration of 10 µM, the effect of the E2-ALG or the E2-ADG on the cells was larger than that of the negative control (the KRB solution alone) as an average value, but not significantly different therefrom and there was also no significant difference between the E2-ALG and the E2-ADG (not shown).

The present inventors have previously reported that fluorescent L-glucose such as 2-NBDLG or CLG are taken up by mouse pancreatic tumor cells or human osteosarcoma cells, which exhibit a malignant feature such as difference in nuclear size, via a specific transport mechanism inhibited by PHT, an aglycon of a polyphenol Phlorizin included in a portion of Rosaceae plants such as apple skin or seeds or cherry leaves (see, Human Cell 29: 37-45, 2016; Human Cell 34: 634-643, 2021; Org. Lett. 18: 1338-1341, 2016). In addition, the previous experimental results by the present inventors suggest that a transport mechanism of these fluorescent L-glucose derivatives into cells is a non-transporter type, probably a channel-like mechanism that does not distinguish between D-glucose derivative and L-glucose derivative (Human Cell 29: 37-45, 2016). Such a channel-like mechanism may be specifically expressed in plasma membranes of malignant tumor cells found in a patient with refractory cancer, and in fact, fluorescent L-glucose such as 2-NBDLG (Human Cell 29: 37-45, 2016) or CLG are hardly taken up into normal cells or tumor cells exhibiting a benign feature (US10001487B2, US10509041B2, EP3130596B1, JP6566348, and ZL201580030346.9). Therefore, the below-described experiments were further conducted.

Whether cell death in MIN6 cells caused by E2-ALG and E2-ADG was via a specific transport mechanism that is inhibited by PHT was analyzed on the same plate. Specifically, the E2-ALG at 1,000 µM was applied to the MIN6 cells in the presence or absence of PHT at 150 µM for 90 minutes, and cytotoxicity caused by the E2-ALG, i.e., whether the PHT significantly reduced a proportion of dead cells expressed as a PI value was statistically tested. The E2-ADG was also tested simultaneously in the same manner on the same plate and both were compared to a control.

Experimental results showed that the PHT eliminated approximately 75% of the cytotoxicity caused by the E2-ALG and the E2-ADG in the MIN6 cells at 11 DIV, i.e., at a time when a number of cells exhibiting a malignant tumor feature had increased (Fig. 3). Such high specificity of inhibition of uptake of the E2-ALG and the E2-ADG into tumor cells by the PHT in 90 minutes, a practical administration time applied in clinical practice, was completely unexpected. This invention overturns the conventional wisdom that has been constructed in the past with regard to fluorescently labeled D-glucose.

To the best of present inventors' knowledge, the E2-ALG and the E2-ADG are a first example of a pair of glucose derivatives that are enantiomers of each other, of which a D form does not have a strong preference for a glucose transporter, and that are selectively taken up by tumor cells exhibiting a malignant tumor cell feature via a non-glucose transporter pathway that is specifically inhibited by Phloretin and thus selectively cause significant cell death in these cells.

### [Discussion]

The above-described results indicate that the E2-ALG and the E2-ADG were selectively taken up by cells exhibiting a malignant tumor feature via the specific mechanism inhibited by the PHT and caused strong cell death in 90 minutes. Compared to the 90-minute administration, the 30-minute administration had a smaller effect on the cells. Referring to relationship between an administration concentration and an effect, a cytotoxic mechanism became apparent between 100 and 1,000 µM in this short-time single-dose study, but a cytotoxic effect could be caused at a lower concentration with longertime observation.

An -SAuP(C₂H₅)₃ group attached at position 2 of L-glucose and D-glucose in E2-ALG and E2-ADG, respectively, is a group attached at position 1 of D-glucose in Auranofin, an FDAapproved anti-rheumatic drug. The Auranofin further has a structure in which all hydrogen atoms in an OH group of glucose are substituted with acetyl groups. The Auranofin has a relatively small side effect and has been reported to have an anticancer activity against ovarian cancer, breast cancer, non-small cell lung cancer, acute lymphocytic leukemia, osteosarcoma, and various other cancers. However, there are many unclear points in its mechanism of action, and clinical trials therefor have been conducted repeatedly for drug repositioning. Recently, it has been reported that Auranofin is effective in nutritional deficit pancreatic cancer cells (Onodera, T. et al. J. Biol. Chem. 295, 16678-16690, 2020) and that Et₃PAuCl alone is cytotoxic to colon cancer cells (ACS Med. Chem. Lett. 8: 997-1001, 2017).

To the best of the present inventors' knowledge, there is no experiment or report describing that, when glucose analog molecules, which were enantiomers of each other and in which D-glucose and L-glucose were similarly substituted at position 2 with the same substituent that exhibited cytotoxicity, were applied to mammalian cells and compared for a property of passing through a glucose transporter or a channel-like transport mechanism (non-glucose transporter type), the D-glucose analog which was substituted at position 2 showed no statistically significant difference from its enantiomer, the L-glucose analog, in a degree of cell death, the D-glucose analog did not pass through the glucose transporter, and both the D-glucose and L-glucose analogs passed through the channel-like transport mechanism and exhibited cytotoxicity. In other words, both E2-ADG and its enantiomer E2-ALG are expected to be a new type of glucose analog with a potential to minimize toxicity to non-cancer cells since they pass through the same channel-like transport mechanism and exhibit cytotoxicity, but do not pass through the glucose transporter that is widely expressed in normal cells. In particular, if the E2-ADG, a D-glucose analog, does not pass through the GLUT which is widely expressed in normal cells almost throughout the body, its side effect on the normal cells may be much smaller than those of a D-glucose analog that passes through the GLUT, and it may be relatively inexpensively supplied because it is a D-glucose analog. Therefore, the E2-ADG is expected to have a medical-economic advantage. On the other hand, the E2-ALG, an L-glucose analog that does not bind to a membrane protein with a D-glucose binding site, has a potential to cause effective regression of cancer with a higher safety even when used at higher concentrations than the D-glucose analog.

### <Administration test using M2-ADG or M2-ALG>

### [Example 6-1] Administration to MIN6 cells early after culture initiation

An administration test was performed in the same manner as in Example 5-1, except that M2-ADG (synthesized in Example 1) was used instead of E2-ADG, and M2-ALG (synthesized in Example 3) was used instead of E2-ALG. However, the M2-ADG or the M2-ALG was administered for 30 minutes after 4 DIV from initiation of culture, and a concentration of the M2-ADG or the M2-ALG was 10 µM, 100 µM, or 1,000 µM.

As a result, as in Example 5-1, there was no statistically significant difference in cellular activity as assessed with Calcein between an M2-ADG administration group and an M2-ALG administration group at any concentration (Fig. 4). In particular, when the concentration is 10 µM or 100 µM, there is no significant difference in cellular activity between the M2-ADG or the M2-ALG and a negative control, KRB alone, with only a slight decrease in an average value. There was also no significant difference in cellular activity between the M2-ADG and the M2-ALG. However, at the concentration of 1,000 µM, a significant decrease in cellular activity was observed in any case of the M2-ADG or the M2-ALG, and there was no significant difference between the two administration groups in an effect of reducing cellular activity (Fig. 4).

On the other hand, occurrence of cell death upon administration of the M2-ADG or the M2-ALG to MIN6 cells was evaluated using a dead cell marker PI, and results are shown in Fig. 5. When the administration concentration was 10 µM, there was no difference in a degree of cell death between either M2-ADG or M2-ALG administration groups and the negative control (Fig. 5). At the administration concentration of 100 µM, an average value increased, but there was still no significant difference from the negative control (Fig. 5). However, clear cell death was observed at the concentration of 1,000 µM, as can be seen from Figure 5. These results clearly differ from those obtained when E2-ALG or E2-ADG was administered for the same period, i.e., 30 minutes (Example 5-1). At none of the above administration concentrations, no statistically significant difference was observed between the M2-ADG administration group and the M2-ALG administration group (Fig. 5).

## Claims

1. A glucose derivative selected from the group consisting of an L-glucose derivative represented by General Formula (1) below and a D-glucose derivative represented by General Formula (2) below: 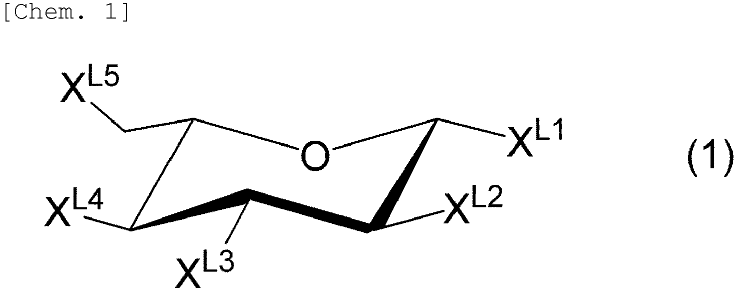
wherein X^{L2} denotes an -SAuR^{L2} group, X^{L1}, X^{L3}, X^{L4}, and X^{L5} each independently denote an -OR^{L1} group, an -NH₂ group, or a fluorine atom,
R^{L2} denotes a ligand, and R^{L1} denotes a hydrogen atom or an organic group. 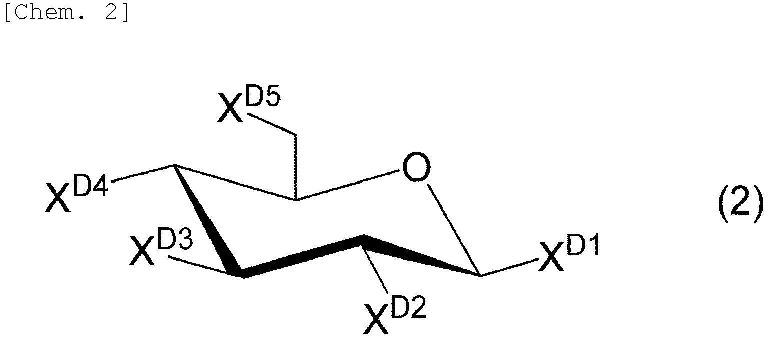
wherein X^{D2} denotes an -SAuR^{D2} group, X^{D1}, X^{D3}, X^{D4}, and X^{D5} each independently denote an -OR^{D1} group, an -NH₂ group, or a fluorine atom,
R^{D2} denotes a ligand, and R^{D1} denotes a hydrogen atom or an organic group.

2. The glucose derivative according to claim 1, wherein X^{L1}, X^{L3}, X^{L4}, and X^{L5} each independently denote an -OR^{L1} group, X^{D1}, X^{D3}, X^{D4}, and X^{D5} each independently denote an -OR^{D1} group.

3. The glucose derivative according to claim 1 or 2, wherein R^{L2} and R^{D2} each independently denote a trialkylphosphine ligand.

4. The glucose derivative according to any one of claims 1 to 3, being the L-glucose derivative.

5. An anticancer agent comprising at least one of the glucose derivatives according to any one of claims 1 to 4.
